# EUROPÄISCHE PATENTANMELDUNG

(11) **EP 1 528 069 A1**
(43) Veröffentlichungstag der Anmeldung: **04.05.2005**
(21) Anmeldenummer: 03405777.8
(22) Anmeldetag: 29.10.2003
(51) Int. Cl.: C08B 30/12, C08L 3/00, C08L 3/02, C08L 3/08, A61K 9/48, A61J 3/07

(54) **Verbesserte Materialien aus Stärke**

(71) Anmelder: SWISS CAPS Rechte und Lizenzen AG, 9533 Kirchberg (CH)
(72) Erfinder: Ménard, Rico, 9533 Kirchbeg (CH); Stolz, Leo, 9608 Ganterschwil (CH); Lutz, Valentin, 9552 Bronschhofen (CH); Brocker, Erich, Dr., 533 Kirchberg (CH)
(74) Vertreter: Welch, Andreas, Dr.

(57) **Zusammenfassung**

Die vorliegende Erfindung betrifft ein Verfahren zur Herstellung eines Materials, umfassend die Schritte der Herstellung einer homogenisierten, thermoplastischen Masse aus einem Gemisch, enthaltend mindestens ein zur Netzwerkbildung befähigtes Polysaccharid, Wasser und mindestens einen Weichmacher, unter erhöhtem Druck und erhöhter Temperatur, des schnellen Abkühlens der so erhaltenen thermoplastischen Masse, und der kontrollierten Zufuhr von Energie zur Durchführung einer gesteuerten Retrogradation. Die vorliegende Erfindung betrifft weiterhin die mit diesem Verfahren erhältlichen Produkte sowie eine Vorrichtung zur Durchführung dieses Verfahrens.

## Beschreibung

Die vorliegende Erfindung betrifft Materialien, vorzugsweise Formkörper aus Polysacchariden wie insbesondere Stärke sowie ein Verfahren zu deren Herstellung.

Formkörper aus biologisch abbaubaren Materialien sind aus Gründen des Umweltschutzes schon seit längerer Zeit von ausserordentlichem Interesse. Als Folge der BSE-Problematik gewinnen insbesondere auch Formkörper zur oralen Einnahme als Nahrungsergänzungsmittel oder pharmazeutische Darreichungsform, wie Kapselhüllen aus gelatinefreiem Material, an Bedeutung.

Hartkapseln aus Stärke sind beispielsweise in der US-4,738,724 beschrieben. Hartkapseln sind zweiteilig aufgebaut. Die beiden Einzelteile werden im Tauchverfahren hergestellt und nach Befüllung zusammengesteckt. Hartkapseln weisen einige Nachteile auf, beispielsweise Probleme hinsichtlich der Dichtheit der Steckverbindung oder das aufwendige und zweistufige Herstellungsverfahren.

Für pumpbare, im weitesten Sinne flüssige bzw. pastöse Füllgüter haben sich deshalb Weichkapseln durchgesetzt, d.h. Kapseln mit einteiliger bzw. verschweisster Kapselhülle. Weichkapseln können in kontinuierlichen, automatisierbaren Verfahren hergestellt werden. Die Herstellung der Kapselhülle und das Füllen derselben geschieht dabei in einem einzigen Arbeitsschritt. In diesen kontinuierlichen, einstufigen Verfahren werden Formteile gefertigt, aus denen die Kapselhülle während und nach dem Füllen durch Verschweissen der Aussenkanten der Formteile zusammengefügt werden. Die Formteilfertigung geschieht entweder mittels auseinander- und zusammengehender Formen, wie z.B. im Norton-Prozess oder mittels rotierender Formwalzen, wie es z.B. im Rotary-Die-Prozess und im Accogel-Verfahren verwirklicht ist ("Die Kapsel" Fahrig/Hofer-Herausgeber, Stuttgart, 1983; Lachmann/Liebermann/Kanig, "The Theory and Practice of Industrial Pharmacy"; Third Edition, Philadelphia 1986). Das Füllen erfolgt mit Hilfe von Dosierpumpen, die eine definierte Menge Wirksubstanz während des Ausstanzens und Verschweissens der Formteile zur Bildung einer einteiligen Kapselhülle abgeben. Das Verschweissen, d.h. die Ausbildung der Nähte erfolgt generell durch Druck und Wärme. Die Herstellkosten sind gegenüber der Herstellung von zweiteiligen Steckkapseln erheblich reduziert.

Ebenso ist das Herstellen von Formkörpern ohne Naht mittels konzentrischer Koextrusion, im einfachsten Fall im Tropfverfahren mittels Schwerkraft (Globex-Verfahren) möglich, wie es beispielsweise allgemein in der WO 00/15173 beschrieben ist.

Der Herstellungsprozess für einteilige Kapseln stellt an das Kapselhüllmaterial eine Reihe von Anforderungen. Eine der Hauptvoraussetzungen ist die Fähigkeit des Kapselhüllmaterials hochelastische "endlose" Bänder mit einer ausreichenden Festigkeit auszubilden. Die Kapselhülle muss sich bei Bedarf im Magendarmtrakt rasch lösen, um die Wirksubstanzen freisetzen zu können. Das Kapselhüllmaterial muss verschweissbar sein. Die Moleküle des die Formteile bildenden Materials, insbesondere die Makromoleküle des Polymeren, sollten sich an der Nahtstelle idealerweise durchdringen, um eine ausreichende Stabilität der Nahtstelle zu gewährleisten.

In der EP-A-1 103 254 ist eine Masse enthaltend homogenisierte Stärke, Wasser und einen organischen Weichmacher beschrieben, welche zur Herstellung von Formkörpern und insbesondere Weichkapseln verwendet werden kann. Zur Herstellung dieser Masse wird eine native oder chemisch modifizierte Stärke mit einem Amylopektingehalt von mindestens 50-Gew.%, bezogen auf das Gewicht der wasserfreien Stärke, zusammen mit Wasser und einem organischen Weichmacher beispielsweise in einem Extruder in eine homogenisierte, thermoplastische Schmelzmasse überführt. Diese Masse kann in Form eines Materialstrangs extrudiert und beispielsweise mittels des Rotary-Die-Prozesses zur Herstellung von Weichkapseln herangezogen werden.

Ein grundsätzliches Problem bei der Verwendung von Stärke als Material für Formkörper liegt in der Tendenz zur Versprödung. Stärke neigt zur Retrogradation. Darunter versteht man eine oft unerwünschte und unkontrollierbare Rekristallisation des Materials, durch welche die besagte Versprödung hervorgerufen wird. Durch die Retrogradation werden verschiedene Materialeigenschaften, unter anderem die Stabilität des Formkörpers oft nachteilig beeinflusst.

Unter Retrogradation im Sinne der vorliegenden Erfindung soll
a) die Bildung von α-helikalen Strukturen aus zwei Amylose-Einheiten aus vollständige hydratisierten Stärkelösungen (vernetzte Stärke),
b) die Bildung von Bündeln mehrerer helikaler Amylose-Doppelstränge zu Kristalliten aus vollständige hydratisierten Stärkelösungen, sowie
c) das Weiterwachsen der kristallinen Zonen (Superhelikale Strukturen) in Amylose- bzw. Amylopektinhaltigen, nur partiell verkleisterten Stärken (vgl. Planchot et al., in Fazier/Donald (Hrsg.), Starch: Structure and Functionality, Royal Society of Chemistry, Special Publication 205, Cambridge 1997, S. 141-152)
verstanden werden.

Die Retrogradation wurde in der US-5,051,271 zur gezielten Beeinflussung der Produkteigenschaften von Nahrungsmitteln auf Stärkebasis eingesetzt. In der US-5,051,271 wird die eingesetzte Stärke vollständig solvatisiert und anschliessend durch Inkubation der so erhaltenen wässrigen Lösung bei unterschiedlichen Temperaturen einer vollständigen Retrogradation unterworfen. Das so erhaltene Produkt weist im wesentlichen keine amorphen Abschnitte mehr auf. Das so erhaltene Produkt ist unter anderem schlechter verdaubar. Das Verfahren bietet einen Zugang zur Herstellung von "resistant starch", welche dietärische Faserwirkung entfalten kann.

In der US-5,292,542 wird ein Verfahren zur Herstellung dehydrierter Kartoffelprodukte wie Pommes Frites vorgeschlagen, bei welchem das stärkehaltige Material unter anderem einer vollständigen Retrogradation unterworfen wird.

In der DE-C1-100 62 848 wird ein Verfahren zur Herstellung von wasserformbeständigen thermoplastischem Stärkematerial beschrieben. Hierbei wird das Stärkematerial zusammen mit einem Destrukturierungsmittel in einem Doppelwellenextruder in ein thermoplastisches Material überführt. Anschliessend wird das Stärkematerial bei hohen Luftfeuchten von mehr als 80% oder in dicht verschlossenen Räumen oder Behältern ohne Luftaustausch für längere Zeit gelagert. Eine kontrollierte Energiezufuhr erfolgt nicht. Unter diesen Bedingungen erfährt das Stärkematerial eine Retrogradation. Gemäss der DE-C1-100 62 848 ist das so erhaltene retrogradierte Produkt besser verarbeitbar und *wasserformbe* *ständig.* Es ist nicht beschrieben, dass dieses Material zur Herstellung spezieller Formkörper wie Weichkapseln verwendet werden kann. Es wird nichts darüber ausgesagt, ob das so erhaltene Material unter Versprödung leidet. Zudem ist das auf diese Weise erhaltene Material wasserformbeständig, d.h. wasserunlöslich. Somit ist dieses Material als Hüllenmaterial zur Herstellung von Kapseln für pharmazeutische Darreichungformen gerade nicht geeignet, da gemäss den Vorgaben der Europäischen Pharmakopoe bei oralen Darreichungsformen eine Löslichkeit der Hülle in Wasser innerhalb von 30 Minuten gewährleistet sein muss.

Das in der DE-C1-100 62 848 beschriebene Verfahren weist einige Nachteile auf. Längere Lagerzeiten sind nicht wünschenswert, da sie einer schnellen Weiterverarbeitung des Produkts im Wege stehen. Insbesondere bei Weichkapseln stellt sich das zusätzliche Problem, dass diese bei längerer Lagerung zum Zusammenkleben und zur Deformation neigen.

Es bestand daher die Aufgabe der vorliegenden Erfindung in der Bereitstellung von Formkörpern aus Stärkematerial, insbesondere von Weichkapseln, welche möglichst gleichbleibende Eigenschaften aufweisen und auf kostengünstige, kontrollierte und schnelle Weise hergestellt werden können.

Überraschend wurde gefunden, dass die vorstehende Aufgabe durch kontrollierte Retrogradation eines Polysaccharids, vorzugsweise eines thermoplastischen Stärkematerials, welches nicht aus vollständig in Wasser oder anderen Lösungsmittteln gelöster Stärke besteht und somit einen Teil der natürlichen superhelikalen Strukturen der nativen Stärke aufweist, mittels kontrollierte Zufuhr thermischer Energie gelöst werden kann.

Die vorliegende Erfindung betrifft somit ein Verfahren zur Herstellung eines Polysaccharide, insbesondere Stärke enthaltenden Materials, umfassend die Schritte:
a) Herstellung einer thermoplastischen Masse aus einem Gemisch, enthaltend mindestens ein zur Netzwerkbildung befähigtes Polysaccharid, Wasser und mindestens einen Weichmacher, unter erhöhtem Druck und erhöhter Temperatur
b) Schnelles Abkühlen der so erhaltenen thermoplastischen Masse
c) Kontrollierte Zufuhr von Energie zur Durchführung einer gesteuerten Retrogradation.

Es hat sich überraschend gezeigt, dass gemäss dem vorstehenden Verfahren hergestellte Materialien wie Formkörper im wesentlichen gleichbleibende physikalische Eigenschaften aufweisen und insbesondere nur noch eine geringe Versprödungstendenz bei nahezu unveränderter Härte zeigen. Diese Materialien beziehungsweise Formkörper sind somit lagerstabil und schnell herstellbar.

Das vorstehende Verfahren kann on-line durchgeführt werden, d.h. die kontrollierte Retrogradation wird in der Regel unmittelbar nach Herstellung des geformten Produkts durchgeführt. Es sind somit keine längeren Lagerzeiten einzuhalten. Das Produkt kann zügig weiterverarbeitet und/oder versandt werden.

Die vorliegende Erfindung wird weiterhin durch die beiliegenden Zeichnungen veranschaulicht. Es zeigen:
- Fig. 1: eine Darstellung verschiedener Strukturen innerhalb eines Polysaccharids, wobei der Typ I ausschliesslich auf kovalenten Bindungen beruhende Strukturen aufweist, der Typ II zusätzliche, auf Wasserstoffbrückenbindungen beruhende Strukturen aufweist, und der Typ III zusätzliche Überstrukturen (Superstrukturen) aufweist, die nicht auf kovalenten Bindungen beruhen;
- Fig. 2: den schematischen Aufbau einer erfindungsgemässen Vorrichtung zur Durchführung einer kontrollierten Retrogradation; und
- Fig. 3: eine graphische Darstellung der Ergebnisse der Untersuchungen gemäss Beispiel 2 (Fig. 3A) und Vergleichsbeispiel 1 (Fig. 3B).

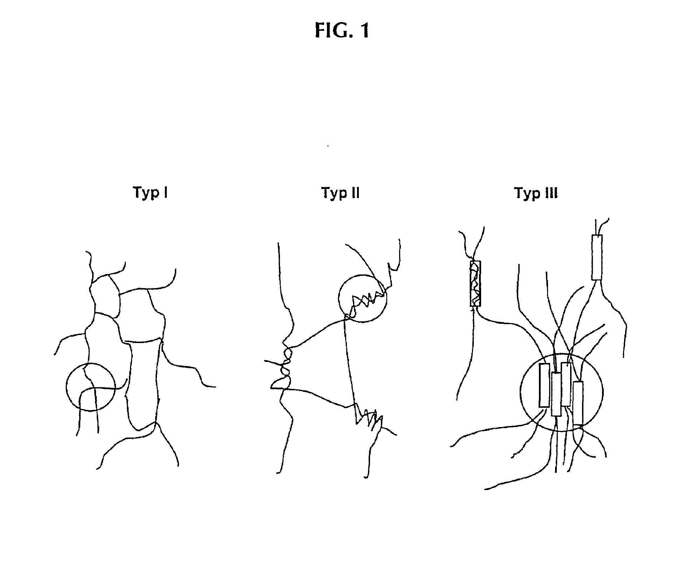

Grundsätzlich können die erfindungsgemässen Produkte auf jedem Polysaccharid basieren, welches zur Netzwerkbildung befähigt ist. Gemäss der vorliegenden Erfindung soll unter einem zur Netzwerkbildung befähigten Material ein derartiges Material verstanden werden, das nicht allein auf kovalenten Bindungen beruhende vernetzte Strukturen ausbilden kann. Das erfindungsgemäss verwendbare Polysaccharid muss Strukturen des in Fig. 1 gezeigten Typs II oder III aufweisen. Diese Strukturen können beispielsweise durch Eintrag von Wärme mindestens graduell "entnetzt" (d.h. zerstört) werden und sich beispielsweise nach Wegfall der Wärmezufuhr erneut ausbilden (Retrogradation). Beide Vorgänge, die Entnetzung und die Retrogradation, können nur stattfinden, wenn eine ausreichende Beweglichkeit der Ketten und eine für den jeweiligen Prozess ausreichende Zeit zur Verfügung steht.

Das erfindungsgemäss bevorzugte Polysaccharid ist Stärke. Unter dem Begriff Stärke sollen native Stärken, unverkleisterte, partiell verkleisterte Stärken oder vollständig verkleisterte Stärken sowie deren zusätzlich chemisch modifizierte Varianten verstanden werden. Unter Verkleisterung wird der Vorgang verstanden, nach welchem oberhalb einer für jede Stärkeart typischen Temperatur in wässrigen Stärkesuspensionen nach Erreichen eines Höchstquellgrades "Lösung", d.h. irreversible Desintegration der Moleküle eintritt. Bei dieser irreversiblen Quellung bis zum 40-fachen des ursprünglichen Volumens erfolgt eine allmähliche Wasseraufnahme und Lösung von Wasserstoffbrückenbindungen, die eine weitere Hydratation bis zur völligen Desintegration des Stärkekorn-Gefüges ermöglicht.

Gemäss der vorliegenden Erfindung sind die in EP-A-1 103 254 beschriebenen Stärken, auf deren diesbezügliche Offenbarung hiermit ausdrücklich Bezug genommen wird, als Hüllmaterial verwendbar. Hierbei handelt es sich vorzugsweise um unverkleisterte Stärke, deren Amylopektingehalt über 50% bezogen auf das Gesamtgewicht der wasserfreien Stärke liegt.

Für die vorliegende Erfindung sind jedoch im weitesten Sinne alle Polyglucane, d.h. 1.4 und/oder 1.6 Poly-α-D-glucane, die mit sich selbst oder anderen Polyglucanen Netzwerkstrukturen bilden können, und/oder Abmischungen zwischen diesen geeignet. Es können somit auch mindestens teilweise verkleisterte Stärken eingesetzt werden, deren Amylosegehalt über 50% bezogen auf das Gesamtgewicht der wasserfreien Stärke liegt.

In einer bevorzugten Ausführungsform ist die Stärke eine unverkleisterte, hydroxypropylierte Stärke. Der Substitutionsgrad (DS = degree of substitution) ist dabei im Bereich von 0.01 bis 0.5, vorzugsweise im Bereich von 0.05 bis 0.25 und noch bevorzugter im Bereich von 0.1 bis 0.15. Insbesondere handelt es sich um hydroxypropylierte Kartoffelstärke.

Erfindungsgemäss am meisten bevorzugt ist die Verwendung einer mindestens teilweise verkleisterten Stärke, deren Amylopektingehalt über 50% bezogen auf das Gesamtgewicht der wasserfreien Stärke liegt und die nach der erfindungsgemässen Verarbeitung zu einer homogenisierten Masse einen Staudinger-Index von wenigstens 40 ml/g, vorzugsweise wenigstens 50 ml/g und noch bevorzugter wenigstens 80 ml/g aufweist.

Gemäss der vorliegenden Erfindung kann das in Schritt a) einzusetzende Gemisch auch andere physikalisch und/oder chemisch modifizierte Biopolymere ausser Stärke enthalten, insbesondere Polysaccharide und pflanzliche Polypeptide. Die Gruppe der physikalisch und/oder chemisch modifizierten Biopolymere umfasst unter anderen Celluloseether und Celluloseester (z-B. Hydroxypropylcellulose, Celluloseacetat), Alginate (Alginsäure und Salze), Carrageenane (lambda, iota, kappa), Agar, Pektine und Pektinderivate aus verschiedenen Früchten (z.B. Apfel, Limonen etc), Galacatomannane (z.B. Guar und Johannisbrotkernmehle), Glucomannane (z.B. Konjac), Arabinogalactane, Pflanzliche Gummis (Acacia gum, Tragant, Karaya, Tamarinde), Gummis aus Mikroorganismen wie Bakterien und Pilzen ( z.B. Xanthan), Aminozuckerderivate (wie Chitosan, Chitin ), Proteine (Casein, Zein, Gluten etc), Dextrine, Maltodextrine, Cyclodextrine, Gellan, Pullulan, und synthetische oder "debranched" Stärke. Weiterhin kann die Hüllmasse auch physiologisch verträgliche synthetische Polymere wie beispielsweise PVP (Polyvinylpyrrolidon), PVA (Polyvinylalkohol), Polyvinylacetat, PLA (Polyessigsäure) oder Acrylester umfassen.

Die zur Herstellung des erfindungsgemässen Produkts eingesetzte Mischung enthält das Biopolymer, vorzugsweise Stärke, vorzugsweise in einem Gewichtsbereich von 45 bis 80 Gew.-% bezogen auf das Gesamtgewicht der Mischung. Die verwendeten Biopolymere, insbesondere die vorstehend beschriebene Stärke, sollten erfindungsgemäss bevorzugt einen Feuchtigkeitsgehalt von 1 bis 30%, vorzugsweise von 15 bis 23% aufweisen.

Das im vorstehenden Verfahren einzusetzende Gemisch umfasst mindestens einen Weichmacher. Die Weichmacher-Komponente muss so gewählt werden, dass eine permanente Plastifizierung des Formkörpers bleibt.

Die organischen Weichmacher werden vorzugsweise ausgewählt aus der Gruppe bestehend aus Polyalkoholen, organischen Säuren, Aminen, Säureamiden und Sulfoxiden sowie Pyrrolidonen. Bevorzugt sind Polyalkohole. Beispiele für erfindungsgemäss verwendbare Weichmacher sind Glycerin, Sirup aus hydrierten, Polyalkohole enthaltenden Stärkeabbauprodukten, wie zum Beispiel Sorbitol, Maltitol, Mannitol, Erythritol, Xylitol, synthetische Polyalkohole wie Propylenglycol, Polyglycerine, Polysorbitane, Polyethylenglycole, Polyethylen-Polypropylen-Polymerisate, oberflächenaktive Stoffe wie z.B. Sorbitanfettsäureester, N-Methylpyrrolidon und Gemische davon.

Der Gesamtweichmachergehalt der eingesetzten Mischung beträgt mindestens 12 Gew.-% bezogen auf das Gewicht der wasserfreien Stärke. In einer bevorzugten Ausführungsform ist der Gehalt des Weichmachers in einem Bereich von 30 Gew.-% bis 60 Gew.-% und noch bevorzugter in einem Bereich von 38 Gew.-% bis 55 Gew.-%, bezogen auf das Gesamtgewicht der Masse.

Der gesamte Wassergehalt der als Ausgangsmaterial eingesetzten Mischung, d.h. die in den Komponenten (Polysaccharid, Weichmacher) enthaltene Menge an Wasser zusammen mit der zusätzlich als Lösungsmittel hinzuzufügenden Menge an Wasser, sollte gemäss der vorliegenden Erfindung in einem Bereich von 6 bis 30 Gew.-% bezogen auf die Gesamtmischung liegen.

Der einzusetzenden Mischung kann je nach erforderlichen Eigenschaften des resultierenden Formkörpers noch ein oder mehrere Zuschlagstoffe in einem Gewichtsbereich von 3,5 Gew.-% bis 15 Gew.-%, bevorzugt von 5 Gew.-% bis 8 Gew.-% bezogen auf das Gesamtgewicht der Mischung zugesetzt werden. Die Zuschlagstoffe können ausgewählt werden aus der Gruppe bestehend aus Carbonaten und Hydrogencarbonaten der Alkali- und Erdalkaliionen, weiteren Zerfallshilfen, Füllstoffen, Farbstoffen und Antioxidantien. Eine Opakisierung der homogenisierten Masse wird bevorzugt mit dem Zusatz von Titandioxid als Füllstoff erreicht. Als Zerfallshilfe für einen schnellen Zerfall der Kapselhülle werden bevorzugt Calciumcarbonat und Amylasen zugesetzt.

In einer bevorzugten Ausführungsform enthält die eingesetzte Mischung zusätzlich ein internes Gleit- und Formtrennmittel, welches ausgewählt ist aus der Gruppe bestehend aus Lecithinen, Mono-, Di- oder Triglyceriden von Speisefettsäuren, Polyglycerinestern der Speisefettsäuren, Polyethylenglycolestern der Speisefettsäuren, Zuckerestern der Speisefettsäuren, und Speisefettsäuren sowie deren Alkali- und Erdalkalisalze sowie Kombinationen davon. Das Gleit- und Formtrennmittel ist in der Mischung bevorzugt in einem Bereich von 0 bis 4 Gew.% bezogen auf das Gesamtgewicht der Mischung enthalten. Vorzugsweise wird es der Mischung in einer Menge von 0,5 bis 2 Gew.% und noch bevorzugter von 0,8 bis 1,5 Gew.% zugesetzt. Vorteilhaft ist das Gleit- und Formtrennmittel ausgewählt aus der Gruppe bestehend aus Glycerinmonostearat und Lecithin. Unter Speisefettsäuren werden die als Säurekomponenten der Triglyceriden natürlicher Fette vorkommenden Monocarbonsäuren verstanden. Sie weisen eine gerade Anzahl von C-Atomen auf und haben ein unverzweigtes Kohlenstoffgerüst. Die Kettenlänge der Fettsäuren variiert von 2 bis 26 C-Atomen. Eine erfindungsgemäss bevorzugte Gruppe dieser Fettsäuren sind gesättigte Fettsäuren.

In einem ersten Schritt wird ein Gemisch aus den vorstehenden Komponenten, vorzugsweise aus Stärke, Wasser und einem Weichmacher, unter kontrollierten Bedingungen in eine thermoplastische Masse überführt. Dieses Verfahren ist beispielsweise in der EP-A-1 103 254 ausführlich beschrieben. Auf deren diesbezüglichen Inhalt wird hiermit ausdrücklich Bezug genommen.

Die Begriffe "thermoplastisch verarbeitbar", "Schmelze", "Sirup" und "amorph" werden in der vorliegenden Anmeldung gemäss der Definition im Römpp Chemie Lexikon, Hrsg: J. Falbe, M. Regitz, 9. Auflage, 1992, Georg Thieme Verlag, Stuttgart, verwendet.

Die Überführung einer Stärke enthaltenden Mischung in den thermoplastischen, vorzugsweise homogenisierten Zustand, ebenso wie die danach folgenden Verarbeitungsschritte, müssen unter Bedingungen erfolgen, die einen unkontrollierten Abbau der Amylose- und Amylopektinmoleküle zu kurzen Bruchstücken und die vollständige Lösung der natürlich vorhandenen Netzstrukturen verhindern. Es muss das Zusammenwirken aller Verarbeitungsparameter wie z.B. Temperatur, Druck, Verweilzeit und Knetleistung, während der verschiedenen Schritte berücksichtigt werden. So kann z.B. auch bei relativ hohen Temperaturen ein weitgehender Abbau der Stärkemoleküle bzw. ein vollständiges decoiling (Aufdrehen der helikalen Strukturen) vermieden werden, wenn die Verweilzeiten und Scherkräfte auf die Masse bei diesen Temperaturen klein gehalten wird.

In einer bevorzugten Ausführungsform übersteigt während der Herstellung einer homogenisierten thermoplastischen Masse die Temperatur der Masse in der Verarbeitungseinrichtung, sowie beim Herstellen des Materialstranges 160°C, bevorzugt 140°C, noch bevorzugter 120°C und am vorteilhaftesten 90°C nicht. Bei 160°C sollte insbesondere auch der Aufschlussvorgang in Schritt a) in weniger als 5 Minuten, bevorzugt weniger als 3 Minuten abgeschlossen sein. Die Verarbeitungseinrichtung ist vorzugsweise ein Extruder.

In einer weiteren bevorzugten Ausführungsform überschreitet die in die Masse durch das Kneten eingebrachte Energie zur Erzeugung einer thermoplastisch verarbeitbaren homogenisierten Masse in Schritt a) bis c) 0,3 kWh/kg, bevorzugt 0,2 kWh/kg und noch bevorzugter 0,175 kWh/kg nicht.

Die Überführung in den thermoplastisch verarbeitbaren Zustand bewirkt eine irreversible Quellung der Stärkekörner, was eine Voraussetzung dafür ist, dass die Masse in den homogenen Zustand überführt werden kann bzw. auch nach dem Abkühlen homogenisiert vorliegt. Durch das vorstehende Verfahren wird weiterhin eine Masse erzeugt, in der im wesentlichen mindestens keine sichtbaren (Stärke-)Kornstrukturen mehr vorhanden sind, jedoch mit beispielsweise wide/small-angle- Röntgenstreuung (WAXS und SAXS) allenfalls noch superhelikale Strukturen nachweisbar sind.

Unter dem Begriff "homogene Masse/Material" bzw. "homogenisierte Masse/Material" soll somit ein Material oder eine Masse verstanden werden, die makroskopisch an jeder Stelle im Material die im wesentlichen gleichen physikalische Eigenschaften (Parameter) aufweist. Zu geringfügigen Abweichungen kann es an den jeweiligen Material- oder Formteiloberflächen durch Aufnahme von Luftfeuchtigkeit kommen. Homogen bzw. homogenisiert liegt die Masse dann vor, wenn unter dem Mikroskop die Anzahl der noch sichtbaren Stärkekörner im Schnitt unter einem Prozent liegt. Dazu wird die Masse im thermoplastischen Zustand abgekühlt, in dünne Scheiben geschnitten und unter dem Lichtmikroskop analysiert.

Eine homogenisierte Masse wird erhalten durch Überführen einer entsprechenden Ausgangsmischung in einen erweichten oder sogar flüssigen, thermoplastisch verarbeitbaren Zustand. Der Grossteil der die Mischung ausmachenden Komponenten kann dabei im geschmolzenen Zustand vorliegen und bei genügend langer Stand- und/oder Misch (Knet-) dauer wird die Masse an jeder Stelle der Schmelze die im wesentlichen gleichen Eigenschaften oder chemische Zusammensetzung haben (homogene Masse). Dieser homogene Zustand wird auch bei und nach Abkühlen des thermoplastischen Zustands beibehalten. Es treten keine Entmischungsvorgänge ein. Dies sorgt für gleichmässige mechanische Eigenschaften des Formkörpers bei Raumtemperatur.

Wie vorstehend erwähnt kann der Wasseranteil der eingesetzten Mischung im erfindungsgemässen Verfahren gezielt und auf dem Fachmann wohlbekannte Weise verändert werden. Die physikalischen Parameter, insbesondere die Viskosität, die vom Wassergehalt abhängen, können so Veränderungen unterworfen werden. Ein zusätzlicher Trocknungsschritt nach Fertigstellung der erfindungsgemässen Formkörper ist somit nicht mehr notwendig. Die Veränderung des Wasseranteils der Mischung kann erfindungsgemäss bevorzugt durch kontrolliertes Ablassen von Wasserdampf in einer Dekompressionszone aus der Verarbeitungsvorrichtung oder durch Einführung von Wasser in einer Einspritzzone in die Verarbeitungsvorrichtung durchgeführt werden. Bei der erfindungsgemäss bevorzugten Doppelschneckenextrusion werden eine oder mehrere sogenannte Entgasungszonen eingesetzt, über die das überschüssige Wasser (aus dem Biopolymer wie beispielsweise der Stärke und/oder dem Weichmacher, beispielsweise dem Polyolsirup) der Schmelze entzogen wird. Durch Einstellen des in der Schmelze wirksamen Unterdrucks (z.B. mit Hilfe eines Frischluftventils zwischen Vakuumpumpe und Extruder) kann der Endwassergehalt des extrudierten Materials gezielt eingestellt werden. Bei festem Gesamtdurchsatz wird zum Beispiel mit einem in den Entgasungszonen wirksamen absoluten Druck von 200 mbar erreicht, dass der Endwassergehalt des Extrudats z.B. 6% beträgt; wird der Druck auf 450 mbar eingestellt, so resultiert ein Endwassergehalt von zum Beispiel 12%.

Gemäss einer bevorzugten Ausführungsform der vorliegenden Erfindung weist die thermoplastische homogenisierte Masse einen Staudinger-Index von wenigstens 40 ml/g, vorzugsweise wenigstens 50 ml/g und noch bevorzugter wenigstens 80 ml/g auf. Hinsichtlich der Erläuterungen zu dem Begriff Staudinger-Index und dessen Bestimmung wird auf den gesamten diesbezüglichen Inhalt der EP-A-1 103 254 verwiesen, der hiermit ausdrücklich in Bezug genommen ist.

Die erhaltene thermoplastische Masse kann insbesondere für Herstellung von Weichkapseln auf allen in der Technik bekannten Anlagen zur Herstellung einteiliger Kapseln verarbeitet werden. Als besonders geeignet haben sich kontinuierliche Anlagen und insbesondere der Rotary-Die-Prozess erwiesen. Die Kapselwand wird dabei aus zwei vorab aus einem Film herausgestanzten Formteilhälften unter Wärmewirkung bevorzugt grösser oder gleich 50°C verschweisst. Zwei "endlose Filme" werden durch zwei benachbarte, in gegenläufigem Sinn rotierenden Rollen oder Walzen mit Aussparungen geführt. Während der Stärkefilm durch den Fülldruck der Füllmasse in die Aussparung gepresst und somit die Kapselhälften geformt werden, wird die pump- und spritzbare Kapselfüllung mittels eines Ventils exakt dosiert und über einen Füllkeil in den Einzugszwinkel der Formwalzen eingebracht. Die Form und Grösse der Kapsel ist somit abhängig von den geometrischen Abmessungen der Aussparungen in den Walzen und dem eindosierten Füllvolumen.

Der Umformungsvorgang des Materialstranges zu einem Formkörper, insbesondere die Umformung eines extrudierten Films in eine einteilige Weichkapsel mit den in der Technik bekannten Verfahren, erfordert Bruchdehnungen des Materialstranges, insbesondere des Films von mindestens 100% im Bereich von 40°C bis 90°C bevorzugt von 60°C bis 80°C. In einer bevorzugten Ausführungsform ist die Bruchdehnung des Materialstranges, insbesondere Films, mindestens 160% und noch bevorzugter mindestens 240%.

Die Festigkeit σₘ des Materialstranges, insbesondere des daraus hergestellten Formkörpers sollte bei 25°C und 60% relativer Luftfeuchtigkeit wenigstens 2 MPa betragen. In einer bevorzugten Ausführungsform ist σₘ grösser oder gleich 3.5 MPa und noch bevorzugter grösser oder gleich 5 MPa. Dieser Wert gewährleistet bei Raumtemperatur eine genügende Stabilität der Kapselhülle (Verpackung, Lagerung, Transportsicherheit und Gebrauch).

Das Befüllen erfolgt jedoch bei erhöhter Temperaturen des Filmes, die einen Fülldruck von nicht mehr als 2 MPa notwendig macht. Dies ist mit einem Young'schen Elastizitätsmodul E von kleiner oder gleich 2 MPa bei Verkapselungstemperatur (40°C bis 90°C) bei der vorliegenden Masse gegeben.

Die vorzugsweise mit Hilfe des Rotary-Die-Verfahrens gebildeten Formkörper können mit einer flüssigen Füllmasse gefüllt werden.

Konsequenterweise soll unter dem Begriff Kapsel deshalb nicht nur die typischen Kapselformen verstanden werden, sondern auch jede andere mögliche Form von "Hüllen", wie z.B. Kugeln, Kissen und Figuren. Bis heute existieren zahlreiche Weiterentwicklungen und Abweichungen von diesem grundlegenden Prinzip.

Die Coextrusion, Beschichtung und das Laminieren des erfindungsgemässen Formkörpers mit Materialien, deren filmbildende Eigenschaft auf synthetischen und/oder natürlichen Polymeren beruht, verschafft zusätzlich Möglichkeiten bestimmte Eigenschaften der Kapselhülle durch eine Mehrschichtfolie zu gestalten,

Insbesondere lässt sich durch den mehrschichtigen Aufbau ein Formkörper herstellen, der auf der Innenseite eine gut schweissbare Beschichtung aufweist, während die Aussenseite derart beschichtet wird, dass eine Retardwirkung des Zerfalls oder eine Magensaftresistenz der Kapsel eintritt.

Die Formkörperhülle hat eine Dicke im Bereich zwischen 0,1 und 2 mm, bevorzugt zwischen 0,2 und 0,6 mm.

Gemäss der vorliegenden Erfindung werden die so hergestellten Formkörper, beziehungsweise die extrudierten Bänder, einer schnellen Abkühlung unterworfen. Damit soll eine bei erhöhten Temperaturen auftretende unkontrollierte Retrogradation verhindert werden. Das Material geht bei der schnellen Temperaturabsenkung zunächst in Bereiche erhöhter Viskosität, durch welche ein "Coiling" oder "Decoiling" entscheidend verlangsamt wird, und bei noch tieferen Temperaturen in einen Glaszustand über. Erfindungsgemäss bevorzugt erfolgt eine Absenkung der Temperatur von der in der Verarbeitungseinrichtung herrschenden Temperatur (bis zu 140°C und bis zu ca. 30 MPa) auf etwa 25°C und 0,13 MPa innerhalb weniger als 3 Minuten. Die Absenkung der Temperatur kann durch gängige Abkühlungsvorrichtungen erreicht werden, beispielsweise durch sogenannte "Chill rolls" (Band) oder durch Überführen der Formkörper in luftgekühlte Behälter.

Anschliessend wird der Formkörper beziehungsweise die für eine Lagerung vorgesehene thermoplastische Masse einer kontrollierten Energiezufuhr ausgesetzt.. Überraschend wurde gefunden, dass hierbei eine kontrollierte Retrogradation dieser Produkte durchgeführt werden kann, durch welche die physikalischen Eigenschaften langanhaltend verbessert werden können. Es ist wichtig zu bemerken, dass unter den erfindungsgemässen Bedingungen in diesem Schritt im wesentlichen keine Trocknung des Formkörpers beziehungsweise der thermoplastischen Masse erfolgt. Es handelt sich ausdrücklich nicht um eine Trocknungsstufe. Gemäss der vorliegenden Erfindung wird dann nicht von einer Trockungsstufe gesprochen, wenn sich der aw-Wert des Formkörpers während der Verfahrensstufe um nicht mehr als 0,1 verringert. Der aw-Wert (die Wasseraktivität eines Systems) ist definiert als die Flüchtigkeit von Wasser aus diesem System geteilt durch die Flüchtigkeit von reinem Wasser. Der aw-Wert entspricht annähernd der Raumfeuchtigkeit (%) geteilt durch 100. Befindet sich ein System bei konstanter Raumfeuchtigkeit im Gleichgewicht mit der Umgebung, d.h. das System nimmt weder Wasser auf noch gibt es Wasser ab, so verändert sich der aw-Wert eines Systems nicht.

Gemäss der vorliegenden Erfindung wird das Material über einen Zeitraum von bis zu 6 Stunden, vorzugsweise von 2 bis 4 Stunden, einer Energiezufuhr ausgesetzt. Die Energiezufuhr kann vorzugsweise durch Zufuhr elektromagnetischer Strahlung im Mikrowellenoder Infrarotbereich erfolgen.

Gemäss einer bevorzugten Ausführungsform werden als Stückgut hergestellte Formkörper der kontrollierten Retrogradation unterworfen. Dies erfolgt vorzugsweise in-line, d.h. unmittelbar nach der Herstellung der Formkörper. Somit können die Formkörper sehr schnell ohne aufwendige und zeitraubende Lagerung direkt in einen versandfertigen Zustand überführt werden.

Gemäss dieser bevorzugten Ausführungsform erfolgt die kontrollierte Retrogradation der Formkörper in einer Vorrichtung, in welcher die Formkörper einer dauernden rotierenden Bewegung unterworfen sind. Bei dieser Vorrichtung kann es sich beispielsweise um einen rotierenden Zylinder handeln.

Die Formkörper vor der kontrollierten Retrogradation neigen zur Verformung aufgrund des Drucks aufliegender Formkörper auf darunter liegende Formkörper. Zudem kann eine unerwünschte Verklebung der Formkörper miteinander auftreten, da die polymere Hüllenmasse aufgrund noch nicht ausreichend gebildeter Vernetzung "fliesst". Diese Probleme werden vermieden, indem man die Formkörper während der kontrollierten Retrogradation einer dauernden rotierenden Bewegung unterwirft. Bevorzugt ist eine Bewegung, bei der die Formkörper mindestens alle 5 Sekunden bewegt werden und dabei eine Bewegung der Formkörper gegeneinander (in der Bewegungsphase) von mindestens 10 cm/s erzeugt. Eine Anlagerung von Formkörpern aneinander sowie ein Übereinanderliegen der Formkörper wird dadurch verhindert.

Gemäss dieser bevorzugten Ausführungsform erfolgt die Energiezufuhr der einer dauernden rotierenden Bewegung unterworfenen Formkörper durch Bestrahlung mit einer Infrarotstrahlvorrichtung, vorzugsweise für einen Zeitraum von bis zu 6 Stunden, bei 400 bis 1200 W/m² und einer Frequenz von 5·10¹³ bis 4·10¹⁴ s⁻¹ (d.h. einer Wellenlänge von 750 nm bis etwa 600 µm), bevorzugt von 1 µm bis 10 µm. Bevorzugt wird die Zufuhr von Infrarotstrahlung mit einer Leistung von 1200 W/m² und einem Strahlungsmaximum bei einer Wellenlänge von 8,5 µm. Wahlweise kann aber auch eine Bestrahlung im Mikrowellenbereich (d.h. mit elektromagnetischer Strahlung einer Wellenlänge von 1 mm bis zu 1 m) erfolgen, vorzugsweise mit Mikrowellen einer Wellenlänge von etwa 100 mm (2000 MHz).

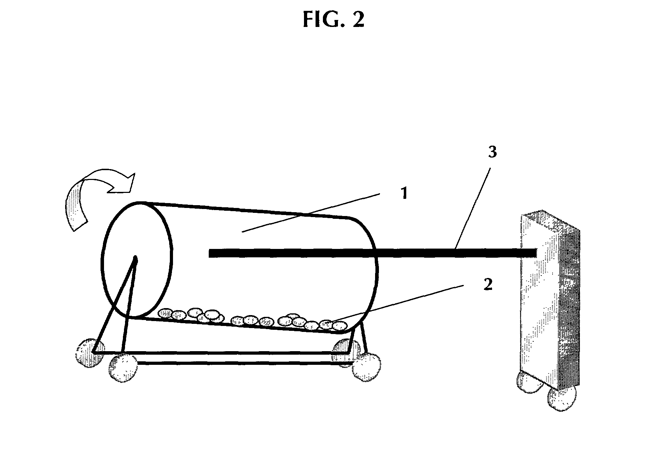

Eine derartige Vorrichtung ist in Fig. 2 gezeigt. Die Formkörper 2 werden unmittelbar nach ihrer Fertigung in eine Vorrichtung 1, beispielsweise einen Drageekessel mit einer Öffnung überführt. Die Vorrichtung 1 kann um einen gegenüber der Vertikale um etwa 60° geneigte Achse rotieren. Ein Infrarotstrahler 3 ist derart ausgebildet, dass er über das Bett aus den Formkörpern 2 geschwenkt werden kann. Obwohl in Fig. 2 der Infrarotstrahler 3 ausserhalb des rotierenden Zylinders 1 angeordnet ist, sind selbstverständlich auch Anordnungen möglich, bei denen der Infrarotstrahler durch eine Öffnung in den rotierenden Zylinder geschoben und über dem Bett aus Formkörpern angeordnet werden kann. Vorzugsweise ist der Infrarotstrahler mit Filtern ausgebildet, welche nur Infrarotstrahlung einer oder mehrerer bestimmter Wellenlängen durchlassen.

Optional kann die Vorrichtung mit einer Heissluftzufuhr ausgestattet sein, um die kontrollierte Retrogradation mit einer Kombination von elektromagnetischer Strahlung und Heissluft durchzuführen.

Gemäss einer besonders bevorzugten Ausführungsform können in der Vorrichtung zur Durchführung einer kontrollierten Retrogradation weitere Verarbeitungsschritte wie eine Trocknung der Formkörper oder eine Entfernung eines gegebenenfalls an den Formkörpern anhaftenden Trennmittels durchgeführt werden. Das führt zu einer weiteren Rationalisierung und Beschleunigung des Gesamtverfahrens.

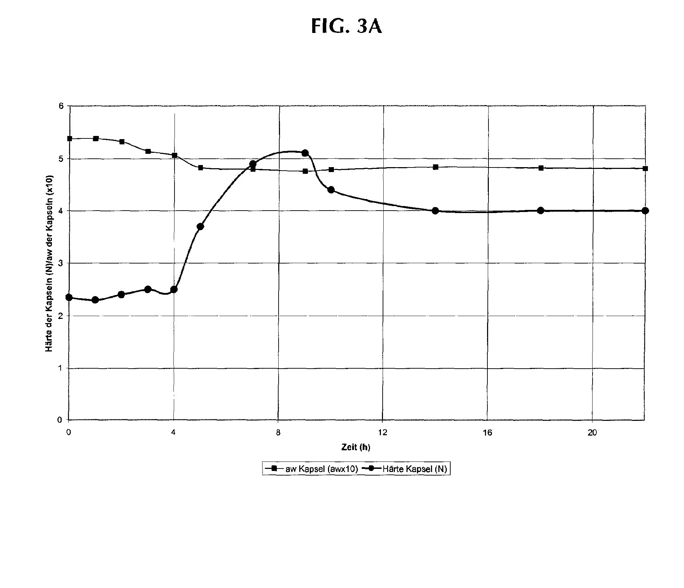

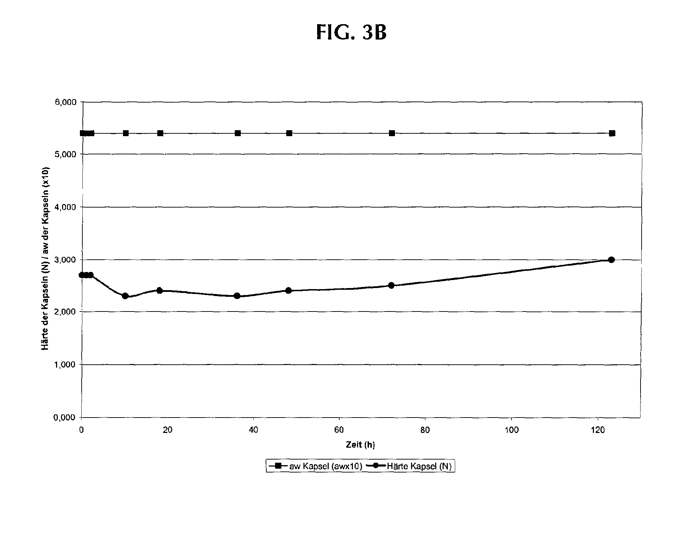

Die einer kontrollierten Retrogradation unterzogenen Formkörper weisen eine deutlich erhöhte Stabilität und Festigkeit auf, welche von Formkörpern, die nicht einer kontrollierten Retrogradation unterworfen werden, wenn überhaupt erst nach einem längeren Zeitraum annähernd erreicht wird. Dies ist in Fig. 3 veranschaulicht.

Das erfindungsgemässe Verfahren eignet sich zur Herstellung verschiedenster Formkörper, jedoch besonders zur Herstellung von Hart- oder Weichkapseln und insbesondere zur Herstellung von Weichkapseln.

Die vorliegende Erfindung wird nachstehend anhand von nicht einschränkenden Beispielen näher erläutert. Soweit nicht anders angegeben, sind alle Mengenangaben in Gewichtsprozent ausgedrückt.

### Beispiele

Eine Weichkapsel kann nur dann ohne Verformung einem üblicherweise bei der Lagerung herrschenden Druck von 10 bis 20 N/m² standhalten, wenn sie eine Härte von mindestens 4 N aufweist, gemessen mit dem Bareiss-Härtetester. Um zu zeigen, dass die jeweilige Änderung der Härte nicht durch eine Trocknung der Kapseln bedingt ist, wurde ebenfalls der Verlauf des aw-Werts der Kapseln während des Vorgangs verfolgt.

### Beispiel 1: Herstellung von Weichkapseln

Eine Hüllmasse für eine Weichkapsel wurde aus einer Mischung folgender Bestandteile hergestellt:

| | |
|---|---|
| Unverkleisterte hydroxypropylierte Stärke | 60 Gew.-% |
| Glycerin | 5 Gew.-% |
| Sorbitolsirup | 19 Gew.-% |
| Maltitolsirup | 15 Gew.-% |
| Glycerinmonostearat | 1 Gew.-% |

Die Mischung wurde in einem Zweiwellenextruder ZSK40 der Firma Werner & Pfleiderer bei einem Temperaturprofil von 90 bis 130°C während maximal 3 Minuten zu einer homogenen Masse geknetet. Die nativen Stärkekörner in der Masse betrugen weniger als 0,1 Gew.-%. Der Wasssergehalt der Masse wurde auf 12% ±1% eingestellt.

Die homogene thermoplastische Masse wurde in 1-Wellenextrudern der Firma Collin zu 0,6 mm dicken Bändern geformt. Anschliessend wurden mittels des Rotary-Die-Prozesses Weichkapseln des Formats 10 minims oval aus 200 mg/Kapsel des so geformten Bandes und 500 mg/Kapsel Sojaöl hergestellt. Die frisch gesiegelten Weichkapseln mit einer Massetemperatur von <40°C wurden in einer Trommel fortlaufend gesammelt, bis sich nach einer Produktionszeit von 50 Minuten ca. 80 Kg Kapseln in der Trommel befanden. Innerhalb dieses Zeitraums befand sich die Trommel in einem Raum mit einer Temperatur von 23°C und 45% RH (Feuchtigkeit). Die Kapseln kühlten in dieser Zeit auf durchschnittlich 25 bis 28°C ab, ohne zu trocknen. Die Kapseln wurden an der Oberfläche mechanisch entölt.

### Beispiel 2: Erfindungsgemässe kontrollierte Retrogradation

Die gemäss Beispiel 1 erhaltenen Weichkapseln wurden für 4 Stunden in einem rotierenden Behälter gemäss Fig. 2 einer IR-Strahlung ausgesetzt und anschliessend für weitere 6 Stunden im rotierenden Behälter bei 22°C und 20% RH (Raumluft) gehalten.

Wie sich aus Fig. 3 A ergibt, wurde die erforderliche Härte von mindestens 4 N bereits nach 10 Stunden erreicht. Während des Vorgangs verringerte sich der aw-Wert von 0,54 auf 0,48. Eine Trocknung fand daher nicht statt. Nach Verbringung in Lagerbehälter und Einstellung des Gleichgewichtes bei aw=0,48 kam es zu keiner Veränderung der Form der Weichkapseln. Die Kapseln waren lagerfähig.

### Vergleichsbeispiel 1

Die gemäss Beispiel 1 erhaltenen Kapseln wurden ohne weitere Behandlung in Kisten mit einer Betthöhe von ca. 10-15 cm gelagert. Wie aus Fig. 3B ersichtlich war auch nach 120 h bei einer Temperatur von 23°C die erforderliche Härte von mindestens 4 N nicht erreicht.

Der Vergleich von Beispiel 2 und Vergleichsbeispiel 1 zeigt, dass sich durch das erfindungsgemässe Verfahren Formkörper wesentlich schneller in einen stabilen Zustand überführen lassen.

## Patentansprüche

1. Verfahren zur Herstellung eines Materials, umfassend die Schritte:
a) Herstellung einer homogenisierten, thermoplastischen Masse aus einem Gemisch, enthaltend mindestens ein zur Netzwerkbildung befähigtes Polysaccharid, Wasser und mindestens einen Weichmacher, unter erhöhtem Druck und erhöhter Temperatur
b) Schnelles Abkühlen der so erhaltenen thermoplastischen Masse
c) Kontrollierte Zufuhr von Energie zur Durchführung einer gesteuerten Retrogradation.

2. Verfahren nach Anspruch 1, **dadurch gekennzeichnet, dass** das Polysaccharid Stärke ist.

3. Verfahren nach Anspruch 1, **dadurch gekennzeichnet, dass** die Stärke ein unverkleisterte, native oder teilverkleisterte Stärke ist.

4. Verfahren nach Anspruch 1, **dadurch gekennzeichnet dass** die Stärke eine unverkleisterte, native oder teilverkleisterte, chemisch modifizierte Stärke ist.

5. Verfahren nach Anspruch 1, **dadurch gekennzeichnet, dass** die Stärke eine mindestens teilweise verkleisterte Stärke mit einem Anteil von mind. 50% Amylose ist

6. Verfahren nach Anspruch 1, **dadurch gekennzeichnet, dass** die Stärke eine mindestens teilweise verkleisterte Stärke mit einem Anteil von mind. 50% Amylopektin ist.

7. Verfahren nach einem der Ansprüche 1 bis 6, **dadurch gekennzeichnet, dass** die homogenisierte thermoplastische Masse einen Staudinger-Index von wenigstens 40 ml/g, vorzugsweise wenigstens 50 ml/g und noch bevorzugter wenigstens 80 ml/g aufweist.

8. Verfahren nach einem der Ansprüche 1 bis 7, **dadurch gekennzeichnet, dass** das in Schritt a) eingesetzte Gemisch Stärke in einem Gewichtsbereich von 45 bis 80 Gew.-% bezogen auf das Gesamtgewicht des Gemisches, mindestens 12 Gew.-% eines Weichmachers, und Wasser enthält.

9. Verfahren nach einem der Ansprüche 1 bis 8, **dadurch gekennzeichnet, dass** der Schritt a) bei einer Temperatur von maximal 140°C während etwa 3 Minuten und einer eingebrachten Energie von maximal 0,3 kWh/kg durchgeführt wird.

10. Verfahren nach einem der Ansprüche 1 bis 9, **dadurch gekennzeichnet, dass** die in Schritt a) erhaltene Masse direkt zu Formkörpern , insbesondere mit Hilfe des Rotary-Die-Verfahrens oder mittels Spritzguss zu Weich-oder Hartkapseln, weiterverarbeitet wird.

11. Verfahren nach einem der Ansprüche 1 bis 10, **dadurch gekennzeichnet, dass** der Schritt c) in einer in einer Vorrichtung erfolgt, in welcher die Formkörper einer dauernden rotierenden Bewegung unterworfen sind.

12. Verfahren nach einem der Ansprüche 1 bis 11, **dadurch gekennzeichnet, dass** die Energiezufuhr mittels eines Infrarotstrahlers erfolgt, der über einem Bett aus den Formkörpern angeordnet ist.

13. Verfahren nach einem der Ansprüche 1 bis 12, **dadurch gekennzeichnet, dass** beim Schritt c) eine Bestrahlung mit Infrarot mit einer Frequenz von 5·10¹³ bis 4·10¹⁴ s⁻¹ und einem Energieeintrag von etwa 400 bis 1200 W/m² über einen Zeitraum von bis zu 6 h erfolgt.

14. Verfahren nach einem der Ansprüche 1 bis 12, **dadurch gekennzeichnet, dass** beim Schritt c) eine Bestrahlung mit Mikrowellen einer Wellenlänge von 100 mm erfolgt.

15. Verfahren nach einem der Ansprüche 1 bis 14, **dadurch gekennzeichnet, dass** das gesamte Verfahren in-line durchgeführt wird.

16. Verfahren nach einem der Ansprüche 11 bis 15, **dadurch gekennzeichnet, dass** in der Vorrichtung zur kontrollierten Retrogradation zusätzlich weitere Verarbeitungsschritte durchgeführt werden.

17. Verfahren nach einem der Ansprüche 1 bis 16, **dadurch gekennzeichnet, dass** Formkörper aus der Gruppe bestehend aus Hartkapseln und Weichkapseln, hergestellt werden.

18. Polysaccharidhaltiges Material, erhältlich nach dem Verfahren gemäss einem der Ansprüche 1 bis 17.

19. Material nach Anspruch 18, **dadurch gekennzeichnet dass** es sich um Formkörper aus Stärke, insbesondere um Hartkapseln oder Weichkapseln handelt.

20. Vorrichtung zur Durchführung des Verfahrens gemäss einem der Ansprüche 1 bis 19, umfassend eine Einheit zur Herstellung einer homogenisierten thermoplastischen Masse, eine Einheit, in welcher die eingeführten Materialien einer dauernden rotierenden Bewegung unterworfen sind, sowie eine Einheit zur kontrollierten Zufuhr von Energie zur Durchführung einer gesteuerten Retrogradation.

21. Vorrichtung nach Anspruch 20, **dadurch gekennzeichnet, dass** die Einheit, in welcher die eingeführten Materialien einer dauernden rotierenden Bewegung unterworfen sind, ein rotierender Zylinder ist.

22. Vorrichtung nach Anspruch 20 oder 21, **dadurch gekennzeichnet, dass** die Einheit zur kontrollierten Zufuhr von Energie ein Infrarotstrahler ist.

23. Vorrichtung nach einem der Ansprüche 20 bis 22, **dadurch gekennzeichnet, dass** die Einheit zur Herstellung einer homogenisierten thermoplastischen Masse ein Extruder ist.

24. Vorrichtung nach einem der Ansprüche 20 bis 23, weiterhin umfassend eine Einheit zur Herstellung von Formkörpern, insbesondere eine Einheit zur Durchführung des Rotary-Die-Verfahrens.

25. Verfahren zur Behandlung eines Polysaccharide, insbesondere Stärke enthaltenden Materials, umfassend den Schritt einer kontrollierten Zufuhr von Energie zur Durchführung einer gesteuerten Retrogradation.
